# EUROPEAN PATENT SPECIFICATION

(11) **EP 1 882 443 B1**
(45) Date of publication and mention of the grant of the patent: **28.01.2015**
(21) Application number: 07013602.3
(22) Date of filing: 11.07.2007
(51) Int. Cl.: A61B 1/273

(54) **Endscope system**
Endoskopsystem
Système endoscopique

(30) Priority: 27.07.2006 JP 2006205209
(43) Date of publication of application: 30.01.2008
(73) Proprietor: OLYMPUS MEDICAL SYSTEMS CORP., Tokyo 151-0072 (JP)
(72) Inventor: Matsui, Raifu, Hachioji-shi Tokyo 192-8512 (JP); Matsuura, Nobuyuki, Hachioji-shi Tokyo 192-8512 (JP); Takase, Seisuke, Hachioji-shi Tokyo 192-8512 (JP); Kimura, Hidenobu, Hachioji-shi Tokyo 192-8512 (JP); Yoshida, Takatoshi, Hachioji-shi Tokyo 192-8512 (JP)
(74) Representative: von Hellfeld, Axel

(56) References cited:
- EP-A- 1 743 569
- JP-A- 2003 135 388
- JP-A- 2004 305 505
- US-A1- 2005 165 272

## Description

The present invention relates to an endoscope system configured to retrieve a capsule endoscope from a patient's body cavity by using an endoscope having a long, thin insertion section.

A capsule-shaped endoscope called a capsule endoscope is known as a means for examining the digestive tract easily. If the digestive tract has a constriction, the capsule endoscope will stop in the constriction. This indicates that the digestive tract is constricted at that point. Having found that the digestive tract is constricted, the doctor can apply an appropriate treatment to the constriction.

As is known in the art, an endoscope is generally used to retrieve the capsule endoscope from the constriction in the digestive tract. In order to retrieve the capsule endoscope, forceps are passed through the accessory channel of the endoscope and manipulated to hold the capsule endoscope. The forceps are then pulled out, retrieving the capsule endoscope from the constriction in the digestive tract. U.S. Pat. Appln. Pub. No. 2004/0133076 A1, for example, discloses a capsule endoscope having a hole in the back so that it may be easily retrieved. The opening of the hole is smaller than the bottom thereof. In other words, the hole has a smaller diameter at the opening than at the bottom. Hence, the hole has a cross section that flares toward the bottom and will therefore be referred to as a flaring hole. Forceps having a distal end that can be expanded and contracted are used to retrieve the capsule endoscope. That is, the distal end is inserted into the flaring hole of the capsule endoscope and expanded, whereby the forceps hold the capsule endoscope. Then, the forceps are pulled, retrieving the capsule endoscope.

Since any capsule endoscope includes hemispherical end parts and a hollow cylinder connecting the end parts, it is hard to hold with forceps. This makes it difficult to retrieve the capsule endoscope from the constriction in the digestive tract by using forceps. Even if the capsule endoscope is held by such forceps as disclosed in U.S. Pat. Appln. Pub. No. 2004/0133076 A1, it may slip from the forceps due to a force produced as it contacts the body wall or as the forceps contact the body wall. Consequently, it may take a long time to retrieve the capsule endoscope, or much skill is required to do so. To hold the capsule endoscope steadily with the forceps as disclosed in U.S. Pat. Appln. Pub. No. 2004/0133076 A1, the flaring hole made in the back of the capsule endoscope must have a larger diameter and must flare more markedly from the opening to the bottom. Even if the capsule endoscope is thus held steadily, it may easily slip from the forceps when it receives a force greater than the force with which it is held by the forceps. This is because almost the entire surface of the capsule endoscope remains exposed while the capsule endoscope is being retrieved.

Document JP 2003-135388 A concerns an endoscope apparatus which allows receiving of a capsule endoscope within a recess of an insertion section of the endoscope by means of suction force.

Document EP 1 743 569 A1 concerns a method for collecting and transporting a medical capsule within an endoscopic apparatus. The endoscopic apparatus includes a sucking pump for sucking the medical capsule to the endoscopic apparatus.

Document US 2005/0165272 A1 concerns an endoscope system having an insertion section to be inserted into a body cavity and a capsule endoscope. In one embodiment, the endoscope system comprises a forceps including a magnet for recovering the capsule endoscope within the body cavity.

Document JP 2004-305505 A concerns an endoscope system for holding and releasing a capsule type endoscope by means of a sucking force.

The present invention has been made to solve the problems described above. An object of the invention is to provide an endoscope system that can easily and reliably retrieve a capsule endoscope.

An endoscope system according to this invention, designed to retrieve a capsule endoscope, includes the features of claim 1.

The invention can be more fully understood from the following detailed description when taken in conjunction with the accompanying drawings, in which:
FIG. 1 is a schematic sectional view of an endoscope system according to a first embodiment of the present invention, showing a hood attached to the distal end of the insertion section of the endoscope and a capsule endoscope inserted in the receptacle of the hood;
FIG. 2 is a schematic diagram showing the configuration of a capsule endoscope to be retrieved by using the endoscope system according the first embodiment;
FIG. 3 is a schematic perspective view showing the endoscope incorporated in the endoscope system according to the first embodiment;
FIG. 4 is a schematic diagram showing the suction mechanism incorporated in the endoscope system according to the embodiment;
FIG. 5A is a longitudinal sectional view showing the suction control valve of the suction mechanism incorporated in the endoscope system according to the embodiment, illustrating the distal end of the insertion section of the endoscope, in which no suction force is exerted;
FIG. 5B is a longitudinal sectional view showing the suction control valve of the suction mechanism incorporated in the endoscope system according to the embodiment, illustrating the distal end of the insertion section of the endoscope, in which a suction force is exerted;
FIG. 6 is a schematic sectional view of an endoscope system according to a second embodiment of the present invention, showing a hood attached to the distal end of the insertion section of the endoscope and a capsule endoscope inserted in the receptacle of the hood;
FIG. 7 is a schematic sectional view of an endoscope system according to a third embodiment of the present invention, showing a receptacle provided in the distal end of the insertion section of the endoscope and a capsule endoscope inserted in the receptacle;
FIG. 8 is a schematic sectional view of an endoscope system according to a fourth embodiment of the present invention, showing a receptacle provided in the distal end of the insertion section of the endoscope and a capsule endoscope inserted in the receptacle;
FIG. 9 is a schematic sectional view of an endoscope system according to a fifth embodiment of the present invention, showing a receptacle provided in the distal end of the insertion section of the endoscope and a capsule endoscope inserted in the receptacle; and
FIG. 10 is a schematic sectional view of an endoscope system according to a sixth embodiment of the present invention, showing a receptacle provided in the distal end of the insertion section of the endoscope and a capsule endoscope inserted in the receptacle.

Best modes of the present invention will be described, with reference to the accompanying drawings.

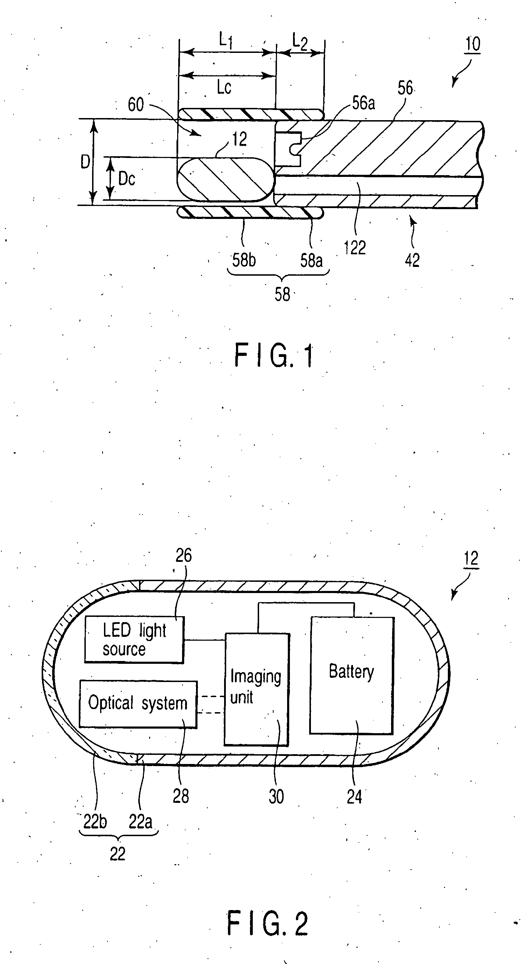

The first embodiment will be described with reference to FIGS. 1 to 5B.

As shown in FIG. 1, the endoscope system 10 according to the first example is designed for use in combination with a capsule endoscope 12 and an endoscope 14 including an elongated insertion section 42. The system 10 is used to retrieve the capsule endoscope 12 from a body cavity of the patient.

The capsule endoscope 12 is of the known type. It incorporates a small camera that includes, for example, a CCD image sensor or a CMOS image sensor.

As shown in FIG. 2, the capsule endoscope 12 includes a capsule-shaped housing 22, a battery (power supply) 24, an LED light source 26, an optical system 28, and an imaging unit 30. The housing 22 contains the battery 24, LED light source 26, optical system 28 and imaging unit 30. The housing 22 includes a main body 22a and a light-transmitting member 22b, which covers the main body 22a. The member 22b is transparent and can therefore allows the passage of light coming from the LED light source 26 to any object, thereby to illuminate the object, and the passage of light coming from the object to the optical system 28.

The battery 24 is arranged deep in the main body 22a. The LED light source 26 and the optical system 28 are arranged side by side and are held in the front part of the main body 22a. The imaging unit 30, which is configured to produce a video signal representing the image of any object that the optical system 28 has formed, is secured between the optical system 28 and the battery 24. The battery 24 is electrically connected to the LED light source 26 and the imaging unit 30 and supplies power to them.

The imaging unit 30 has, for example, a CCD sensor (not shown) that is arranged in axial alignment with the optical system 28. The CCD sensor can therefore convert any image coming through the optical system 28, into the video signal as it performs photoelectric conversion. The imaging unit 30 is configured to transmit video data by radio from the housing 22. That is, the imaging unit 30 transmits the image formed by the CCD image sensor, by radio from the housing 22 (i.e., the patient).

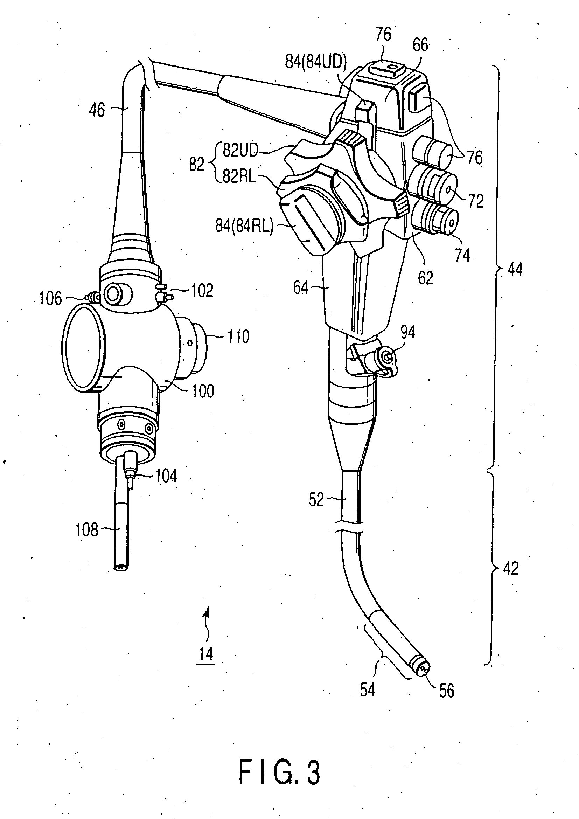

As shown in FIG. 3, the endoscope 14 includes a long, thin insertion section 42, an operation section 44, and a universal cord 46. The operation section 44 is provided at the proximal end of the insertion section 42. The universal cord 46 extends from the operation section 44.

The insertion section 42 includes a flexible tube part 52, a bending part 54, and a rigid distal part 56. The bending part 54 is secured to the distal end of the flexible tube part 52. The rigid distal part 56 is secured to the distal end of the bending part 54. The proximal end of the flexible tube part 52 is coupled to the operation section 44. In the rigid distal part 56 there are arranged an observation optical system, an imaging element, an illumination optical system, an air/water nozzle, a forceps outlet, and the like (not shown).

As shown in FIG. 1, a hood 58 is attached to the rigid distal part 56, pushed onto the rigid distal part 56 from the distal end of the insertion section 42. The hood 58, which can be removed from the rigid distal part 56, is used to retrieve the capsule endoscope 12.

As shown in FIG. 1, the hood 58 includes a connection part 58a and a projecting part 58b. The connecting part 58a can be mounted on the outer circumferential surface of the rigid distal part 56 and secured thereto. The projecting part 58b is integrally formed with the connecting part 58a. The projecting part 58b, which extends from the distal end of the rigid distal part 56, has length L₁ that is almost equal to the length L_{C} of the capsule endoscope 12, which is measured in the axial direction. The hood 58 has an inside diameter D greater than the outside diameter D_{C} of the capsule endoscope 12. The outside diameter D_{C} of the capsule endoscope 12 is, for example, about 5 to 11 mm, and the inside diameter D of the hood 58 is D_{C} + 0.5 to 1.5 mm. Thus, the capsule endoscope 12 can be contained in the receptacle 60 that is a space defined by the distal end of the rigid distal part 56 and the inner circumferential surface of the hood 56.

The projecting part 58b may have its length L₁ and inside diameter D changed to various values, in accordance with the length L_{C} and outside diameter of the capsule endoscope 12. In other words, one of hoods 58 having various sizes is selected in accordance with the specific size of the capsule endoscope 12 used and is secured to the rigid distal part 56. The length L2 of the connecting part 58a is enough to prevent the part 58a from slipping from the distal end of the insertion section 42.

As shown in FIG. 3, the operation section 44 includes a main unit 62, a grip 64, and a switch cover 66.

The main unit 62 includes a suction control valve 72, an air/water valve 74, and remote switches 76. The valve 72, valve 74 and switches 76 are arranged, for example, side by side. The remote switches 76 are provided, in part, inside the switch cover 66.

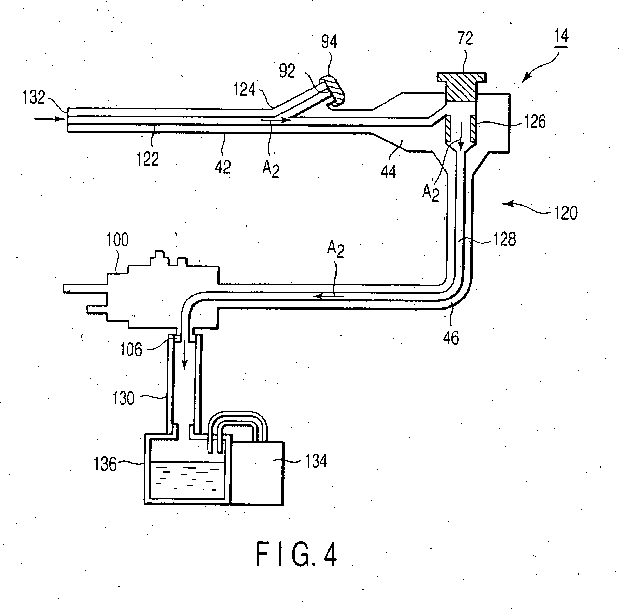

The suction control valve 72 shown in FIGS. 3 and 4 may be operated to switch a suction tubular path 120, which will be described later. More precisely, the suction control valve 72 is operated to switch the tubular path 120 from a suction state to a non-suction state, or vice versa. In the suction state, the tubular path 120 draws liquid medicine, blood and water from the body cavity of the patient. In the non-suction state, the tubular path 120 does not draw medicine, blood or water. As shown in FIG. 3, the air/water valve 74 is provided to supply liquid for washing the objective lens or air for blowing the liquid from the objective lens. One remote switch 76 may be operated to perform a desired process on any video signal produced by the above-mentioned imaging element. The other remote switches 76 may be operated to, for example, magnify any image observed through the endoscope 14 and to extract (or photograph) the image.

On the operation section 44 there are provided angle knobs 82 (i.e., first bending knob 82UD and second bending knob 82LR) and bending-setting levers 84 (i.e., first engage lever 84UD and second engage lever 84LR). The knobs 82UD and 82LR and the levers 84UD and 84LR are made of hard resin.

The first bending knob 82UD is operated to bend the bending part 54 of the insertion section 42 in upward-downward direction. The second bending knob 82LR is operated to bend the bending part 54 in leftward-rightward direction. The first engage lever 84UD is operated to set the first bending know 82UD in a desired position. That is, the first engage lever 84UD is used to hold the bending part 54 in a desired bent state, either upward or downwards. The second engage lever 84LR is operated to set the second bending knob 03LR in a desired position. That is, the second engage lever 84LR is used to hold the bending part 54 in a desired bent state, either leftward or rightwards.

The grip 64 of the operation section 44, which is provided near the insertion section 42, has a forceps port 92 that communicates with the suction tubular path 120. The path 120 is shown in FIG. 4 and will be described later. A forceps plug 94 is removably attached to the forceps port 92. While attached to the forceps port 92, the forceps plug 94 closes the forceps port 92. That is, the forceps plug 94 closes the forceps port 92 as long as no forceps need to be inserted from the forceps port 92.

The universal cord 46 is covered with a sheath made of, for example, polyurethane resin. A connector 100 is attached to that end of the universal cord 46, which is remote from the operation section 44. The connector 100 is made of hard resin.

The connector 100 has a water-tank connecting cap 102, a suction cap 106, a light guide 108, and an electrical connector portion 110. The cap 102 is made of metal and connected to a water tank (not shown). An air pipe 104 is made of metal and connected to an air pump (not shown). Thus, when the air/water valve 74 is operated, air or water can be supplied toward the distal end of the insertion section 42. As shown in FIG. 4, a suction tube 130 is connected at one end to the suction cap 106. The other end of the suction tube 130 is connected to a suction bottle 136, which in turn is connected to a suction pump 134 (receptacle mechanism).

The light guide 108 can be connected to the connector receptacle of a light source (not shown). Therefore, the illumination light produced by the light source can travel from the connector receptacle and pass through the light guide 108 before it is emitted from the illumination optical system that is provided in the rigid distal part 56.

The electrical connector portion 110 is arranged on a side of the connector 100. A cable (connection cord) is connected to the electrical connector portion 110 and to a video processor (not shown). Any electrical signal output from the imaging element (not shown) can thereby be supplied to a monitor. The monitor can therefore display the image supplied to the imaging element through the observation optical system 56a provided in the rigid distal part 56 of the insertion section 42.

As shown in FIG. 4, the section tubular path 120 includes an upstream suction channel 122, a branching tube 124, a suction cylinder 126, a downstream suction channel 128, the suction cap 106 (already mentioned), and the suction tube 130 (already mentioned).

The upstream suction channel 122 is provided partly in the insertion section 42 and partly in the operation section 44. The upstream suction channel 122 communicates, at the distal end, with a suction port (forceps outlet port) 132 that is provided in the distal end of the insertion section 42. The branching tube 124 communicates with the forceps port 92 that is provided in the operation section 44. Thus, the upstream suction channel 122 serves as an accessory channel as well.

The downstream suction channel 128 is provided partly in the operation section 44 and partly in the universal cord 46. The suction cap 106 is attached to the connector 100, at that end of the downstream suction channel 128, which is remote from the operation section 44.

The suction tube 130 is connected at one end to the suction cap 106 and at the other end to the suction pump 134 that has a suction bottle 136. In other words, the suction tube 130 connects the suction cap 106 to the suction bottle 136 of the suction pump 134.

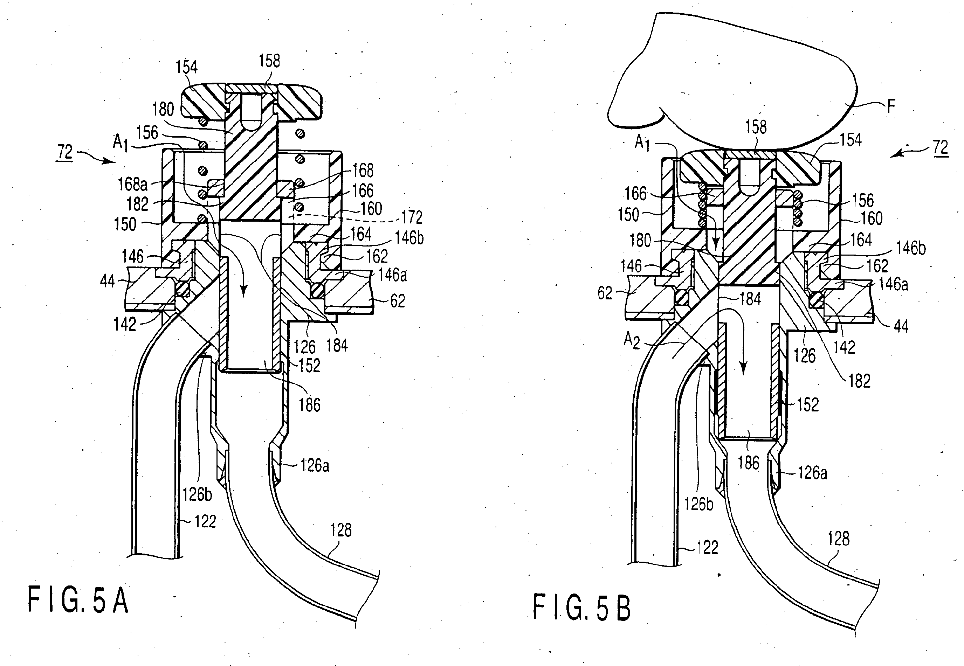

As shown in FIG. 5A, the suction cylinder 126, in which a piston 152 can slide as will be described later, is secured to the main unit 62 of the operation section 44 (see FIG. 3). On the outer circumferential surface of the suction cylinder 126, an O-ring 142 is mounted, sealing the interior of the main unit 62 from the exterior thereof in a watertight fashion. The suction cylinder 126 has a first open part 126a and a second open part 126b. The first open part 126a is the lower part of the cylinder 126, which is provided in the main unit 62, extends in the axial direction of the cylinder 126 and opens at the lower end. The second open part 126b is located near the first open part 126a and has an axis deviating from that of the cylinder 126. To the second open part 126b, the upstream suction channel 122 shown in FIG. 4 is fixed at the proximal end.

A cylinder cap 146 made of metal such as stainless steel is removably fastened to the top of the suction cylinder 126 with, for example, screws. The cylinder cap 146 includes a first flange 146a and a second flange 146b. The first flange 146a is made almost flush with the outer surface of the cylinder cap 146. The second flange 146b outwardly projects from the main unit 62.

The suction control valve 72 includes a holding part 150, a piston 152, a button 154, and a spring 156.

The holding part 150 is shaped like a hollow cylinder and can therefore be mounted on the suction cylinder 126 of the main unit 62 and also on the cylinder cap 146. The piston 152 is arranged to slide along the axis of the cylinder cap 146 shaped like a hollow cylinder. The piston 152 can slide on the inner circumferential surface of the cylinder 126. The button 152 is secured to the top of the piston 152.
The spring 156 is used, spacing the button 154 from the holding part 150 by a prescribed distance. As shown in FIG. 5B, when the button 154 is pushed by a doctor's finger F, the piston 152 is moved down into the cylinder 126. The button 154 has an index 158. The index 158 helps the user to distinguish the suction control valve 72 from the air/water valve 74 and the like. The index 158 has a particular pattern or color that indicates the function of the suction control valve 72, i.e., the control of suction.

The holding part 150 includes a hollow cylindrical main body 160, a projection 162, a partition 164, an extension 166, and a protrusion 168.

The main body 160 has an inside diameter, which is almost equal to or a little larger than the outside diameter of the second flange 146b of the cylinder cap 146.

The projection 162 is a ring projecting from the lower end of the inner circumferential surface of the main body 160 toward the axis of the main body 160.
The projection 162 can move inwardly beyond the second flange 146a and be fitted to the cylinder cap 146. Once the projection 162 has engaged with the second flange 146a, the main body 160 is prevented from slipping from the suction cylinder 126.

The partition 164 extends toward the axis of the partition 164. Hence, the second flange 146b of the suction cylinder 126 abuts, at the upper surface, the partition 164 when the main body 160 is attached to the suction cylinder 126. In other words, the partition 164 is mounted on the second flange 146a.

On the middle part of the partition 164, a pair of extensions 166 are provided, extending upwards along the axis of the main body 160. These extensions 166 are opposed to each other. A leakage port 172 is provided adjacent to the extensions 166. A ring-shaped projection 168 is provided at the upper ends of the extensions 166 and extends into the extensions 166.

The inner circumferential surface of the ring-shaped projection 168 defines a piston hole 168a, in which a piston shaft 180 is fitted to slide. The stepped part 182 of the piston 152, which will be described later, abuts the ring-shaped projection 168 from below. That is, that part of the piston shaft 180, which lies below the stepped part 182, has an outside diameter a little smaller than the inside diameter of the partition 164.

The upper surface of the partition 164 serves as a spring support that supports the lower end of the coil spring 156.

As shown in FIGS. 5A and 5B, the piston 152 has the piston shaft 180. The piston shaft 180 has a step 182 made in the circumferential surface and at the middle part. The upper and lower parts of the piston shaft 180, above and below the step 182, respectively, differ in diameter. The upper part has a smaller diameter than the lower part. The piston shaft 180 has a transverse hole 184, just below the step 182. The hole 184 extends at right angles to the axis of the piston shaft 180. The piston shaft 180 has a longitudinal hole 186, just below the transverse hole 184. The longitudinal hole 186 extends in the axial direction of the piston shaft 180, from the transverse hole 184 to the lower end of the piston shaft 180.

As shown in FIG. 5A, while the button 164 remains unpushed, the transverse hole 184 is at the position where it opens to the suction cylinder 126. The leakage port 172, which lies adjacent to the extensions of the holding part 150, is provided on the same side as the transverse hole 184.

The button 154 is mounted on the upper end of the piston shaft 180. The index 158 is secured to the top of the piston 152. The lower surface of the button 154 serves as the spring support that supports the upper end of the coil spring 156.

The coil spring 156 is mounted on the projection 168 provided in the holding part 150. The lower end of the spring 156 is supported on (or abuts) the upper surface of the partition 164. The upper end of the spring 156 is supported on (or abuts) the lower surface of the button 154. Compressed to some extent, the spring 156 biases the piston 152 toward the upper end of the piston shaft 180 while the button 164 remains unpushed, pushing the step 182 of the piston 152 onto the projection 168. That is, the spring 156 keeps the button 154 spaced from the partition 164 of the main body 160.

In this embodiment, the hood 58 is removably attached to the rigid distal part 56. Instead, it may be removably fastened to the secured to the rigid distal part 56 with, for example, screws.

How the endoscope system 10 according to this embodiment is operated will be explained.

The capsule endoscope 12 is inserted into the patient's digestive tract through the mouth. The light emitted from the LED light source 26 is applied through the light-transmitting member 22b that is transparent. The light illuminates the interior of the digestive tract. The light reflected from any object in the digestive tract is supplied through the light-transmitting member 22b to the optical system 28, which forms an image of the object. The imaging unit 30 converts the image into a video signal, which is transmitted outside the patient.

The capsule endoscope 12 may be caught in a constriction, if any, in the digestive tract. To retrieve (or remove) the capsule endoscope 12 from the digestive tract and ultimately from the patient, the hood 58 is attached to the rigid distal part 56 of the insertion section 43 of the endoscope 14. It is preferably that the projecting part 58b of the hood 58 has length L₁ that is almost equal to the length L_{C} of the capsule endoscope 12.

The suction pump 134, which is connected to the end of the downstream suction channel 128 as shown in FIG. 4, is then driven. The suction bottle 136 and the suction tube 68 are depressurized. As a result, the downstream suction channel 128 is depressurized, too.

While the button 154 of the suction control valve 72 remains unpushed as shown in FIG. 5A, air A₁ is drawn into the downstream suction channel 128 now depressurized, through the space between the inner circumferential surface of the main body 160 and the piston shaft 180, the leakage port 172 of the main body 160, the transverse hole 184 of the piston shaft 180 and the longitudinal hole 186 of the piston shaft 180. The air A₁ is therefore drawn into the suction pump 134 via the suction bottle 136 and discharged from the suction pump 134.

The proximal end of the upstream suction channel 122 is disconnected from the first open part 126a (downstream suction channel 128) and the second open part 126b (upstream suction channel 122) by the lower part of the piston shaft 180, which lies below the transverse hole 184. The upstream suction channel 122 is not depressurized. Hence, no suction force acts at the distal end of the upstream suction channel 122 (see FIG. 4).

As the suction pump 134 is so driven, the insertion section 42 with the hood 58 attached to its distal end is guided to the constriction in the digestive tract. The doctor searches the interior the tract for the capsule endoscope 12, while observing the endoscopic image. On finding the capsule endoscope 12, the doctor moves the capsule endoscope 12 into the hood 58 attached to the distal end of the insertion section 42.

The doctor then operates the button 154 of the suction control valve 72 in order to hold the capsule endoscope 12 in the receptacle 60 by suction before pulling the insertion section 42. How the suction control valve 72 is operated to hold the capsule endoscope 12 in at the distal end of the upstream suction channel 122 will be explained below.

When the button 154 of the suction control valve 72 is pushed by the doctor's finger F, the airflow of the air A₁ in the space between the inner circumferential surface of the main body 160 and the piston shaft 180 is blocked as shown in FIG. 5B at the outer circumferential surface of the piston shaft 180 and the inner circumferential surface of the cylinder 126.

The second open part 126b at the proximal end of the upstream suction channel 122 comes to communicate with the transverse hole 184 of the piston shaft 180. As a result, the air A₂ is drawn from the upstream suction channel 122 via the longitudinal hole 186 of the piston shaft 180 and the first open part 126a into the downstream suction channel 128 that has been depressurized.

Then, the body liquid and the like in the receptacle 60 are drawn from the rigid distal part 56 of the insertion section 43 of the endoscope 14 into the downstream suction channel 128 that has been depressurized, through the upstream suction channel 122, the second open part 126b of the cylinder 126, the transverse hole 184 of the piston shaft 180 and the longitudinal hole 186 of the piston shaft 180. At this time, the body fluid and the like are stored in the suction bottle 136 and the air A₂ and other gases drawn at the same time are drawn and discharged by the suction pump 134.

The capsule endoscope 12 drawn into the receptacle 60 is attracted, by a suction force, to the suction port 132 that is provided in the distal end of the insertion section 42. With the suction control valve 72 depressed, attracting the capsule endoscope 12, the insertion section 42 of the endoscope 14 is pulled from the digestive tract. The capsule endoscope 12 can thereby be easily retrieved.

When the distal end of the insertion section 42 that is being pulled leaves the patient, the button 154 of the suction control valve 72 is released from the doctor's finger F. The downstream suction channel 128 and the upstream suction channel 122 are disconnected as shown in FIG. 5A, and the downstream suction channel 128 comes to communicate with the outside via the transverse hole 184. Air A₁ is drawn into the suction bottle 136 and thence into the suction pump 134. The air A₁ is then discharged from the suction pump 134. Any suction force no longer acts on the capsule endoscope 12. Thus, the capsule endoscope 12 falls from the suction port 132 by its weight. The capsule endoscope 12 is thus retrieved from the patient.

In this embodiment, the capsule endoscope 12 is retrieved by using a suction force. Nonetheless, grasping forceps or the like may be guided from the forceps plug 94 through the upstream suction channel 122 and caused to protrude from the suction port 132. The doctor may hold the capsule endoscope 12 with the grasping forceps and move the same into the receptacle 60 and may then hold the capsule endoscope 12 in the receptacle 60 by suction, thereby to retrieve the same. To hold the capsule endoscope 12 by suction in the receptacle 60, an accessory that applies a magnetic force or a suction force at the distal end may be guided through the upstream suction channel 122 to the position where the capsule endoscope 12 is located.

As has been described, the present embodiment achieves the following advantages.

In order to retrieve a capsule endoscope 12 from a digestive tract, the hood 58 defining the receptacle 60 is attached to the distal end of the insertion section 42 of the endoscope 14. Then, the capsule endoscope 12 is moved into the receptacle 60 and drawn by suction. The capsule endoscope 12 is thus attracted to the distal end of the insertion section 42 of the endoscope 14. The insertion section 42 is pulled, whereby the capsule endoscope 12 is retrieved. To retrieve the capsule endoscope 12, it suffices to attach the hood 58 to the distal end of the insertion section 42 of the endoscope 14. Therefore, preparation for the retrieval of the capsule endoscope 12 can be made easily, and the capsule endoscope 12 can be retrieved quickly.

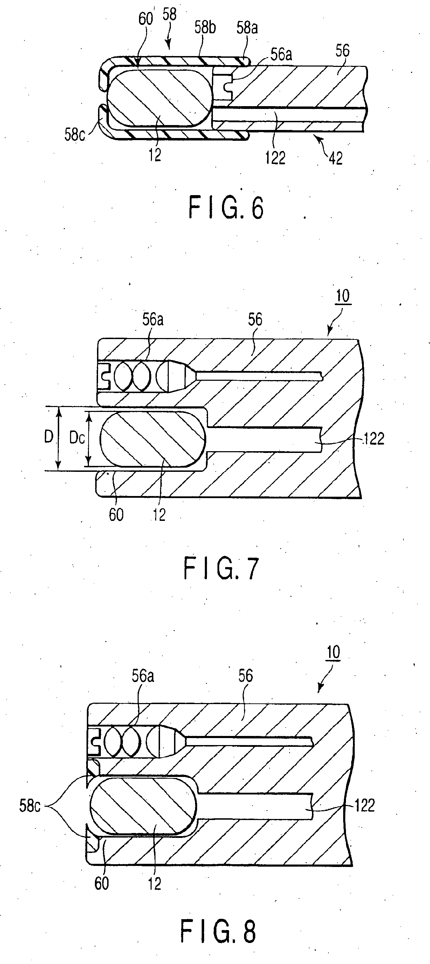

The second example will be described with reference to FIG. 6. This embodiment is a modification of the first embodiment. The components identical to those of the first embodiment are designated at the same reference numbers in FIG. 6 and will not be described in detail.

As shown in FIG. 6, the hood 58 attached to the distal end of the insertion section 42 of an endoscope 14 includes a connection part 58a, a projecting part 58b, and a receptacle inlet port (valve element, valve-holding mechanism) 58c. The projecting part 58b has an inside diameter, which is larger than the outside diameter of the capsule endoscope 12. The receptacle inlet port 58c is a hollow cylinder that is, at the distal end (opposite to the connection part 58a), rounded and shaped like a flange so a capsule endoscope 12 may easily enter the receptacle 60 and have difficulty coming out of the receptacle 60 (This allows the capsule endoscope 12 to enter the receptacle 60 and prevents it from slipping from the receptacle 60.).

When the capsule endoscope 12 is pushed, at either end, onto the receptacle inlet port 58c in order to hold the capsule endoscope 12 in the receptacle 60, the receptacle inlet port 58c is deformed or bent toward the distal end of the insertion section 42. As a result, the capsule endoscope 12 enters the receptacle 60. Once the capsule endoscope 12 has been received as whole in the receptacle 60, the receptacle inlet port 58c assumes its initial shape, with the aid of its elasticity. This prevents the capsule endoscope 12 from slipping out of the receptacle 60.

While the capsule endoscope 12 is abutting the receptacle inlet port 58c, the button 154 of the suction control valve 72 on the operation section 44 of the endoscope 14 may be pushed. In this case, a suction force is applied to the receptacle 60 through the upstream suction channel 122 and the suction port 132. The suction force draws the capsule endoscope 12 toward the interior of the receptacle 60. This helps to insert the capsule endoscope 12 into the receptacle 60.

While the insertion section 42 of the endoscope 14 is being pulled from a body cavity, no large force is exerted to the receptacle inlet port 58c. Therefore, the insertion section 42 can be pulled from the patient, with the capsule endoscope 12 prevented from slipping out of the receptacle 60, even if the suction force is no longer applied to the capsule endoscope 12.

The receptacle inlet port 58c may not be shaped like a flange. For example, it may be composed of a plurality of claws, e.g., two or three claws projecting from the distal end of the projecting part 58b toward the axis of the hood 58.

The projecting part 58b may have an inside diameter close to the outside diameter of the capsule endoscope 12 as shown in FIG. 6. Then, the projecting part 58b or the receptacle inlet port 58c may reduce the view field of the observation optical system 56a of the insertion section 42 when the endoscope 14 is inserted into the body cavity. Because of this, it is desirable that the hood 58 be transparent.

The third example will be described with reference to FIG. 7. The third embodiment is a modification of the first embodiment. The components identical to those of the first embodiment are designated at the same reference numbers in FIG. 7 and will not be described in detail.

As shown in FIG. 7, no hood is attached to the distal end of the insertion section 42 of the endoscope according to this embodiment. A receptacle 60 is made in the rigid distal part 56 of the insertion section 43 of the endoscope 14. The receptacle 60 is shaped like a recess, in which a capsule endoscope 12 can be held. The receptacle 60 therefore communicates with the upstream suction channel 122 provided in the insertion section 43.

The receptacle 60 has a diameter D that is a little larger than the outside diameter D_{C} of the capsule endoscope 12.

To draw the capsule endoscope 12 into the receptacle 60, the button 154 of the suction control valve 72 is operated, applying a suction force in the receptacle 60 while the capsule endoscope 12 has its either end inserted into the receptacle 60. Then, the suction force draws the capsule endoscope 12 into the receptacle 60. The insertion section 42 of the endoscope 14 is pulled from the body cavity, while the section force is still applied in the receptacle 60. The capsule endoscope 12 is thereby retrieved from the patient.

The capsule endoscope 12 can be drawn into the receptacle 60 from the distal end of the rigid distal part 56 to various depths so that the capsule endoscope 12 may be held in part (for example, about half the entire length) or in its entirety, in the receptacle 60. Hence, the receptacle 60 only needs to be deep enough to receive at least one part of the capsule endoscope 12, provided that it can hold the capsule endoscope 12 by suction.

The fourth example will be described with reference to FIG. 8. The fourth embodiment is a modification of the third embodiment. The components identical to those of the third embodiment are designated at the same reference numbers in FIG. 8 and will not be described in detail.

As shown in FIG. 8, a receptacle inlet port (valve element) 58c shaped like a ring is provided at the distal end of the rigid distal part 56 of the insertion section 43 and communicates with the receptacle 60 made in the rigid distal part 56. The receptacle inlet port 58c is made of material that can elastically deform. The inside diameter of the receptacle inlet port 58c is smaller than the outside diameter of the capsule endoscope 12. Preferably, the receptacle 60 is deeper than the capsule endoscope 12.

When the capsule endoscope 12 is pushed, at either end, at the receptacle inlet port 58c to be inserted into the receptacle 60, the receptacle inlet port 58c is deformed or bent toward the distal end of the insertion section 42. Thus, as the capsule endoscope 12 is further pushed, it enters the receptacle 60.
When the capsule endoscope 12 is entirely received in the receptacle 60, the receptacle inlet port 58c assumes its initial shape by virtue of its elasticity. The receptacle inlet port 58c then prevents the capsule endoscope 12 from slipping out of the receptacle 60.

When the capsule endoscope 12 is placed at the receptacle inlet port 58c, the button 154 of the suction control valve 72 provided on the operation section of the endoscope 14 may be pushed. Then, a suction force is applied to the receptacle 60 through the upstream suction channel 122 and the suction port 132. The suction force draws the capsule endoscope 12 into the receptacle 60. This helps to set the capsule endoscope 12 in the receptacle.

A large force is scarcely exerted on the receptacle inlet port 58c in order to pull the insertion section 42 of the endoscope 14 from a body cavity of the patient. Hence, the capsule endoscope 12 can be easily retrieved, without slipping out of the receptacle 60, even if no suction force is applied to the capsule endoscope 12 held in the receptacle 60.

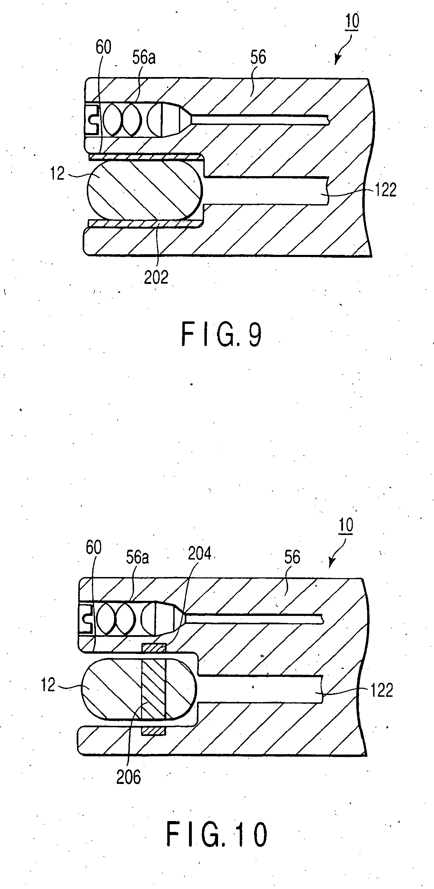

The fifth example will be described with reference to FIG. 9. The fifth embodiment is a modification of the third embodiment, too. The components identical to those of the third embodiment are designated at the same reference numbers in FIG. 9 and will not be described in detail.

As shown in FIG. 9, an adhesive cylinder (holding mechanism) 202 is provided, by coating, on the inner circumferential surface of the receptacle 60. Once a capsule endoscope 12 has been drawn into the receptacle 60, it is adhered, at the outer circumferential surface, to the adhesive cylinder 202. This prevents the capsule endoscope 12 from slipping out of the receptacle 60 made in the endoscope 14.

If a suction force is used to hold the capsule endoscope 12 in the receptacle 60 as explained in conjunction with the first embodiment, the adhesive cylinder 202 need to have such an adhesive force as will assist the suction force. If the outer circumferential surface of the capsule endoscope 12 contacts the inner circumferential surface of the adhesive layer 202, the suction force produced when the suction control valve 72 is operated can act on the capsule endoscope 12. This helps to hold the capsule endoscope 12 in the receptacle 60.

Although not shown in any drawings, the receptacle inlet port 58c shown in FIG. 8 and described in connection with the fourth embodiment may be provided at the entrance to the receptacle 60.

The sixth example will be described with reference to FIG. 10. The sixth embodiment is a modification of the third embodiment, too. The components identical to those of the third embodiment are designated at the same reference numbers in FIG. 9 and will not be described in detail.

FIG. 10 shows, a permanent magnet (holding mechanism) 204 is embedded in the inner circumferential surface of the receptacle 60, and a permanent magnet 206 is mounted on the main body 22a of a capsule endoscope 12. Since a suction force is used to hold the capsule endoscope 12 in the receptacle 60 as explained in conjunction with the first embodiment, the permanent magnets 202 and 204 need to have such an adhesive force as will assist the suction force. In particular, the permanent magnet 206, which is mounted on the main body 22a of the capsule endoscope 12, preferably has a small magnetic force so that electrical noise, which is produced when the capsule endoscope transmits video data as it is guided in a digestive tract, may be as little as possible.

The magnetic forces of the permanent magnets 204 and 206 helps to draw the capsule endoscope 12 into the receptacle 60 when the button 154 of the suction control valve 72 is operated, applying a suction forced on the capsule endoscope 12.

In the first and second embodiments, the hood 58 defines a receptacle 60. In the third to sixth embodiments, a recess is provided in the rigid distal part 56 and used as receptacle 60. The hood 58 and the recess may, of course, be combined. In other words, the hood 58 and the recess may constitute a receptacle 60.

In the embodiments described above, suction is used to draw a capsule endoscope 12 into the receptacle 60. Instead, grasping forceps (not shown) or the like may be guided through the upstream suction channel 122 to the capsule endoscope 12 and may be manipulated to hold the capsule endoscope 12 and move it into the receptacle 60.

## Claims

1. An endoscope system (10) for retrieving a capsule endoscope (12) comprising:
an endoscope (14) which includes:
an insertion section (42) having a distal end (56) and a proximal end and configured to be inserted into a body cavity of a patient;
an operation section (44) arranged at the proximal end of the insertion section;
a receptacle (60) which is provided in the distal end of the insertion section (42) of the endoscope (14) and configured to hold the capsule endoscope (12) in order to retrieve the same;
a suction mechanism configured to hold the capsule endoscope (12) in the receptacle (60); and
an observation optical system (56a) provided at the distal end (56) of the insertion section (42),
**characterized in that** the endoscope (14) further comprises
a hood (58) attached to the distal end (56) of the insertion section (42), the hood (58) comprising a connection part (58a), a receptacle inlet port (58c), and a projecting part (58b) connecting the connection part (58a) with the receptacle inlet port (58c), wherein
the connecting part (58a) is mounted on the outer circumferential surface of the distal part (56) of the insertion section (42) and is secured thereto, and
the receptacle inlet port (58c) is elastic and composed of a plurality of claws projecting from the distal end of the projecting part (58b) toward the axis of the hood (58).

2. The endoscope system (10) according to claim 1, wherein the insertion section (42) of the endoscope (14) includes an accessory insertion channel (122), and the receptacle (60) is configured to communicate with the accessory insertion channel (122).

3. The endoscope system (10) according to claim 1, wherein the suction mechanism includes:
a suction control valve (72) provided on the operation section (44);
a suction pump (134) connected to the suction control valve; and
a channel (122, 128) connecting the distal end (56) of the insertion section (42) of the endoscope (14) and the suction control valve (72).

4. The endoscope system (10) according to claim 1, wherein the hood (58) is transparent.

## Patentansprüche

1. Endoskopsystem (10) zum Zurückholen eines Kapselendoskops (12) aufweisend:
ein Endoskop (14), das enthält:
einen Einführungsabschnitt (42) mit einem distalen Ende (56) und einem proximalen Ende, wobei der Einführungsabschnitt (42) dazu eingerichtet ist, in eine Körperhöhle eines Patienten eingeführt zu werden;
einen Betätigungsabschnitt (44), der an den dem proximalen Ende des Einführungsabschnitts angeordnet ist;
eine Aufnahme (60), die an dem distalen Ende des Einführungsabschnitts (42) des Endoskops (14) bereitgestellt und dazu eingerichtet ist, das Kapselendoskop (12) zu halten, um es zurückzuholen;
einen Saugmechanismus, der dazu eingerichtet ist, das Kapselendoskop (12) in der Aufnahme (60) zu halten; und
ein optisches Beobachtungsystem (56a), das an dem distalen Ende (56) des Einführungsabschnitts (42) bereitgestellt ist,
**dadurch gekennzeichnet, dass** das Endoskop (14) ferner aufweist
eine Haube (58), die an dem distalen Ende (56) des Einführungsabschnitts (42) angebracht ist, wobei die Haube (58) aufweist: ein Verbindungsteil (58a), eine Haubeneinlassöffnung (58c) und ein hervorstehendes Teil (58b), das das Verbindungsteil (58a) mit der Haubeneinlassöffnung (58c) verbindet, wobei
das Verbindungsteil (58a) an der äußeren Umfangsfläche des distalen Teils (56) des Einführungsabschnitts (42) angebracht und daran befestigt ist, und
die Haubeneinlassöffnung (58c) elastisch und aus einer Mehrzahl von Klauen gebildet ist, die von dem distalen Ende des hervorstehenden Teils (58b) zu der Achse der Haube (58) hervorstehen.

2. Endoskopsystem (10) nach Anspruch 1, bei dem der Einführungsabschnitt (42) des Endoskops (14) einen Zusatzeinführungskanal (122) enthält, wobei die Aufnahme (60) dazu eingerichtet ist, mit dem Zusatzeinführungskanal (122) zu kommunizieren.

3. Endoskopsystem (10) nach Anspruch 1, bei dem der Saugmechanismus enthält:
ein Saugsteuerungsventil (72), das an dem Betätigungsabschnitt (44) bereitgestellt ist;
eine Saugpumpe (134), die mit dem Saugsteuerungsventil verbunden ist; und
einen Kanal (122, 128), der das distale Ende (56) des Einführungsabschnitts (42) des Endoskops (14) und das Saugsteuerungsventil (72) verbindet.

4. Endoskopsystem (10) nach Anspruch 10, bei dem die Haube (58) transparent ist.

## Revendications

1. Système (10) d'endoscope destiné à récupérer un endoscope de type capsule (12) comprenant :
un endoscope (14) qui comprend :
une section d'insertion (42) comportant une extrémité distale (56) et une extrémité proximale et configurée pour être insérée dans une cavité de corps d'un patient ;
une section de commande (44) agencée au niveau de l'extrémité proximale de la section d'insertion ;
un réceptacle (60) qui est prévu dans l'extrémité distale de la section d'insertion (42) de l'endoscope (14) et configuré pour maintenir l'endoscope de type capsule (12) afin de récupérer ce dernier ;
un mécanisme d'aspiration configuré pour maintenir l'endoscope de type capsule (12) dans le réceptacle (60) ; et
un système optique d'observation (56a) prévu au niveau de l'extrémité distale (56) de la section d'insertion (42),
**caractérisé en ce que** l'endoscope (14) comprend en outre
un capuchon (58) fixé à l'extrémité distale (56) de la section d'insertion (42), le capuchon (58) comprenant une partie de connexion (58a), un orifice d'entrée (58c) de réceptacle, et une partie de projection (58b) connectant la partie de connexion (58a) avec l'orifice d'entrée (58c) de réceptacle, dans lequel
la partie de connexion (58a) est montée sur la surface circonférentielle externe de la partie distale (56) de la section d'insertion (42) et est fixée à celle-ci, et
l'orifice d'entrée (58c) de réceptacle est élastique et composé d'une pluralité de griffes faisant saillie depuis l'extrémité distale de la partie de projection (58b) vers l'axe du capuchon (58).

2. Système (10) d'endoscope selon la revendication 1, dans lequel la section d'insertion (42) de l'endoscope (14) comprend un canal (122) d'insertion d'accessoires, et le réceptacle (60) est configuré pour communiquer avec le canal (122) d'insertion d'accessoires.

3. Système (10) d'endoscope selon la revendication 1, dans lequel le mécanisme d'aspiration comprend :
une soupape de commande d'aspiration (72) prévue dans la section de commande (44) ;
une pompe d'aspiration (134) connectée à la soupape de commande d'aspiration ; et
un canal (122, 128) connectant l'extrémité distale (56) de la section d'insertion (42) de l'endoscope (14) et la soupape de commande d'aspiration (72).

4. Système (10) d'endoscope selon la revendication 1, dans lequel le capuchon (58) est transparent.
